# EUROPEAN PATENT SPECIFICATION

(11) **EP 0 479 210 B1**
(45) Date of publication and mention of the grant of the patent: **31.05.1995**
(21) Application number: 91116704.7
(22) Date of filing: 30.09.1991
(51) Int. Cl.: C07K 14/645, A61K 38/00

(54) **GIP analogues and use thereof**
GIP-Analoge und deren Verwendung
Analogues du GIP et leur utilisation

(30) Priority: 05.10.1990 JP 266438/90
(43) Date of publication of application: 08.04.1992
(73) Proprietor: SANWA KAGAKU KENKYUSHO CO., LTD., Nagoya, Aichi-ken (JP)
(72) Inventor: Kurono, Masayasu, c/o Sanwa Kagaku Kenkyusho Co., Nagoya, Aichi-ken (JP); Mitani, Takahiko, c/o Sanwa Kagaku Kenkyusho Co., Nagoya, Aichi-ken (JP); Takahashi, Haruo, c/o Sanwa Kagaku Kenkyusho Co., Nagoya, Aichi-ken (JP); Unno, Ryoichi, c/o Sanwa Kagaku Kenkyusho Co., Nagoya, Aichi-ken (JP); Suzuki, Tomoo, c/o Sanwa Kagaku Kenkyusho Co., Nagoya, Aichi-ken (JP); Hayashi, Yuji, c/o Sanwa Kagaku Kenkyusho Co., Nagoya, Aichi-ken (JP); Kobayashi, Yohei, c/o Sanwa Kagaku Kenkyusho Co., Nagoya, Aichi-ken (JP); Ishii, Yoko, c/o Sanwa Kagaku Kenkyusho Co., Nagoya, Aichi-ken (JP); Sawai, Kiichi, c/o Sanwa Kagaku Kenkyusho Co., Nagoya, Aichi-ken (JP)
(74) Representative: Wächtershäuser, Günter, Prof. Dr.

(56) References cited:
- EP-A- 0 269 072
- PROCEEDINGS OF THE NATIONAL ACADEMY OF SCIENCES OF USA. vol. 84, 1 October1987, WASHINGTON US pages 7005 - 7008; J TAKEDA ET AL.: 'sequence of anintestinal cdna encoding human gastric inhibitory polypeptide precursor'
- FEBS LETTERS. vol. 172, no. 2, 1 July 1984, AMSTERDAM NL pages 142 - 148; A JMOODY ET AL.: 'the isolation and sequencing of human gastric inhibitory peptide(gip)'

## Description

The present invention relates to a GIP analogue, namely a novel polypeptide with biological activities as in GIP (Gastric Inhibitory Polypeptide) and use thereof.

The GIP analogue according to the invention can be used as an effective ingredient for medicines, especially for curing diabetes.

The GIP is one of peptide hormones, exists in stomach, intestines, pancreas and other organs, and firstly isolated by Brown, J. C. et al from porcine duodenum ["Can. J. Physiol.", Vol. 47, page 113 - 114 (1969)].

While, human GIP was isolated by Varverde, I. et al who also determined a structure thereof ["FEBS Lett.", Vol. 172, pages 142 - 148 (1984)]. According to descriptions given in the literature, the human GIP is polypeptide coding following 42 amino acids and has molecular weight of about 5000. The GIP was considered to be a member of secretin family, since the amino acid sequence thereof is analogous with the secretin, VIP (Vasoactive Intestinal Polypeptide), glucagon and the like. 
Structure of the native GIP :
5 10
Tyr-Ala-Glu-Gly-Thr-Phe-Ile-Ser-Asp-Tyr-Ser-Ile-Ala-Met-
15 20 25
Asp-Lys-Ile-His-Gln-Gln-Asp-Phe-Val-Asn-Trp-Leu-Leu-Ala-
30 35 40
Gln-Lys-Gly-Lys-Lys-Asn-Asp-Trp-Lys-His-Asn-Ile-Thr-Gln
Each of said porcine and human GIPs had been extracted from animal organ and showed physiological activities which are generally classified into actions accelerating gastric juice secretion and insulin secretion. Among them, it has been known that the action accelerating insulin secretion remarkably increases, as a concentration of blood sugar rises up. In view of this phenomenon, the GIP has been expected as it may become an effective ingredient for curing a certain diabetes. Namely, it is preferable for insulin-independent patients, whose B cell remains, to lie in such a physical state that insulin secretion is particularly accelerated, when the concentration of their blood sugar lies higher level.

The GIP according to the prior arts has been obtained through extraction from animal organ tissue of pork, beef or human being or through a chemical synthesis, and thus it was quite difficult to obtain the same in large amount. Namely, any of GIP has not actually been applied for clinical use, due to its poor productivity, in spite of that an effectiveness thereof as the effective ingredient for medicines to cure diabetes has been expected.

An essential object of the invention is to provide a GIP analogue with biological activities in same level with or higher than native GIP, by chemically or fermentationally synthesizing a polypeptide with a GIP like structure, cleaving the resulting polypeptide with cyanogen bromide to make into another polypeptide coding homoserine (inclusive of homoserine-lactone) at C-terminal and GIP structure, and if necessary carrying out a simple chemical reaction thereon.

The present inventors had already proposed a recombinant plasmid with an insert of DNA clone of human GIP precursor to make possible a large scale production of human GIP by utilizing the biotechnology [Jap. Pat. Appln. No. Sho 61 - 282812 corresponding to EP-0269072(A2) and USSN 616712 which is a continuation of USSN 124646]. Therefore, final object of the invention is to provide the GIP analogue as an effective ingredient for medicines to cure diabetes and with a reasonable price, by combining the techniques given in the literature with those of the present invention to prepare the GIP analogue with high efficiency.

The inventors have energetically studied and investigated to find out firstly a fact that on a polypeptide obtained by a fermentation method or through a chemical synthesis, with a GIP like structure but coding an amino acid residue other than methionine at 14-position, a polypeptide obtained after cleaving treatment of the former polypeptide with cyanogen bromide codes homoserine (inclusive of homoserine-lactone) at C-terminal. Then, they have found another fact that the resulting polypeptide has biological activities compatible to or higher than those of native GIP to establish the invention.

The problems in the prior arts, therefore, dissolve by a GIP analogue which codes an amino acid sequence shown by Formula (1) of 
Tyr-Ala-Glu-Gly-Thr-Phe-Ile-Ser-Asp-Tyr-Ser-Ile-Ala- X -
-Asp-Lys-Ile-His-Gln-Gln-Asp-Phe-Val-Asn-Trp-Leu-Leu-Ala-
Gln-Lys- Y
wherein X is an amino acid residue other than Met, and Y is
a) homoserine (inclusive of homoserine-lactone and shall be referred to as "Hse"),
b) homoserine amide (HseNH₂),
c) Gly-Lys-Lys-Asn-Asp-Trp-Lys-His-Asn-Ile-Thr-Gln-Hse or
d) Gly-Lys-Lys-Asn-Asp-Trp-Lys-His-Asn-Ile-Thr-Gln-HseNH₂,
to attain the objects.

The ground of that the symbol X at 14th position amino acid residue is stated as the residue of amino acid other than Met (methionine) lies in that if X is Met residue, the desired polypeptide with the GIP like activities can not be obtained, since a cleavage will occur at Met position in 14th position, when the polypeptide is treated with cyanogen bromide.

The GIP analogue according to the invention can be made into a double-stranded DNA in accordance with conventional techniques. The double-strand DNA may be cloned to insert the same into a vector such as a plasmid to transform a host such as Escherichia coli which shall be cultured to express the GIP analogue in large amount.

The invention will now be further explained with reference to Examples, Test Example on pharmacological activity and Medicine Preparation Example, which shall refer to drawings, wherein
Fig. 1 is a graph showing results of measurement on glucose dependent insulin secretion activity, which was carried out in accordance with an experiment using isolated pancreatic cells of rat and by selecting, as test samples, GIP analogues obtained by Example 1 and as control sample, a chemically synthesized GIP; and
Fig. 2 is a graph similar to that of Fig. 1, excepting that GIP analogues obtained by Example 2 were selected as test samples.

The Examples are concerned to the preparation of GIP analogues, wherein 14-position amino acid residue is leucine residue different from methionine residue in native GIP, but please note that other 14-position amino acid residue exchanged GIP analogues, wherein the amino acid residue at 13-position is that other than methionine residue, can be prepared in the manner similar to that as described in the Examples.

### Example 1

### [Synthesis of GIP analogues shown by Formulae (1)-a and (1)- b]

In the first place, a polypeptide coding the following amino acid sequence was synthesized with use of a peptide synthesizer (Type 430A marketed by Applied Biosystems Co.) 
Tyr-Ala-Glu-Gly-Thr-Phe-Ile-Ser-Asp-Tyr-Ser-Ile-Ala-Leu-
Asp-Lys-Ile-His-Gln-Gln-Asp-Phe-Val-Asn-Trp-Leu-Leu-Ala-
Gln-Lys-Met-Ala-Ser
A purification of the synthesized polypeptide was carried out by subjecting to HPLC with use of a » Bondasphere C-18 column (19mm x 15cm) marketed by Waters Co.

The resulting purified sample (10mg) was taken and dissolved in 70% formic acid solution (30ml). After added 50mg of cyanogen bromide, the solution was kept for 24 hours at 37°C to cause a reaction. Then, distilled water (200ml) was added to the reaction mixture and lyophilized to remove the formic acid and cyanogen bromide. The resulting material was subjected again to HPLC under the following conditions, with use of » Bondasphere C-18 column (19mm x 15cm) marketed by Waters Co. to purify the same.
- Elute: : Linear gradient of 20% to 70% acetonitrile in 0.1% trifluoroacetic acid (30 minutes),
- Flow rate: : 7.0ml/min.

Fractions in main peak part on the HPLC were recovered and lyophilized. Through said procedures, said amino acid sequence has been cleaved at methionine residue part at 31st position and it is modified into homoserine residue at C-terminal to provide a polypeptide shown by Formula(1)-a and with GIP like activities {[Leu¹⁴]-GIP(1-30)-Hse}.

If the polypeptide is further treated with an acid (for instance, in 0.1N HCl at 30°C for 3 hours) and then lyophilized, the homoserine residue at C-terminal can be modified to homoserine-lactone residue in the level of 70% or more.

The polypeptide with the homoserine-lactone residue at C-terminal was collected through HPLC and lyophilized. The resulting dried powder was treated with 10% ammonia in dimethylformamide solution at room temperature for 24 hours to provide a polypeptide shown by Formula (1)-b and with GIP like activities {[Leu¹⁴]-GIP(1-30]-HseNH₂}.

### Example 2

### [Synthesis of GIP analogues shown by Formulae (1)-c and (1)-d]

In the first place, a polypeptide coding the following amino acid sequence was synthesized with use of a peptide synthesizer (Type 430A marketed by Applied Biosystems Co.) 
Tyr-Ala-Glu-Gly-Thr-Phe-Ile-Ser-Asp-Tyr-Ser-Ile-Ala-Leu-
Asp-Lys-Ile-His-Gln-Gln-Asp-Phe-Val-Asn-Trp-Leu-Leu-Ala-
Gln-Lys-Gly-Lys-Lys-Asn-Asp-Trp-Lys-His-Asn-Ile-Thr-Gln-
Met-Ala-Ser
A purification of the synthesized polypeptide was carried out by subjecting to HPLC with use of a » Bondasphere C-18 column (19mm x 15cm) marketed by Waters Co.

The resulting purified sample (10mg) was taken and dissolved in 70% formic acid solution (30ml). After added 50mg of cyanogen bromide, the solution was kept for 24 hours at 37°C to cause a reaction. Then, distilled water (200ml) was added to the reaction mixture and lyophilized to remove the formic acid and cyanogen bromide. The resulting material was subjected again to HPLC under the following conditions, with use of » Bondasphere C-18 column (19mm x 15cm) marketed by Waters Co. to purify the same under the conditions same with those in Example 1.

Fractions in a main peak part on the HPLC were recovered and lyophilized. Through said procedures, said amino acid sequence has been cleaved at methionine residue part at 43rd position and it is modified into homoserine residue at C-terminal to provide a polypeptide shown by Formula(1)-c and with GIP like activities {[Leu¹⁴]-GIP-Hse}.

A part of the resulting polypeptide was taken and checked with use of a peptide sequencer marketed by Applied Biosystems Co. to confirm that the initially synthesized polypeptide is cleaved at correct position coding methionine residue part at C-terminal and there is the homoserine residue.

If the polypeptide is further treated with an acid (for instance, in 0.1N HCl at 30°C for 3 hours) and then lyophilized, the homoserine residue at C-terminal can be modified to homoserine-lactone residue in the level of 70% or more.

The polypeptide with the homoserine-lactone residue at C-terminal was collected through HPLC and lyophilized. The resulting dried powder was treated with 10% ammonia in dimethylformamide solution at room temperature for 24 hours to provide a polypeptide shown by Formula (1)-d and with GIP like activities {[Leu¹⁴]-HseNH₂}.

### Biological Activity Test Example

### [Measurement of glucose dependent insulin secretion activity using isolated spleen cells of rat]

A measurement on glucose dependent insulin secretion activity was carried out in accordance with an experiment using isolated pancreatic cells of rat ["Endocrinology", Vol. 99, page 780 (1976)] and by selecting, as test samples, GIP analogues obtained by the Examples and as control sample, a chemically synthesized GIP.

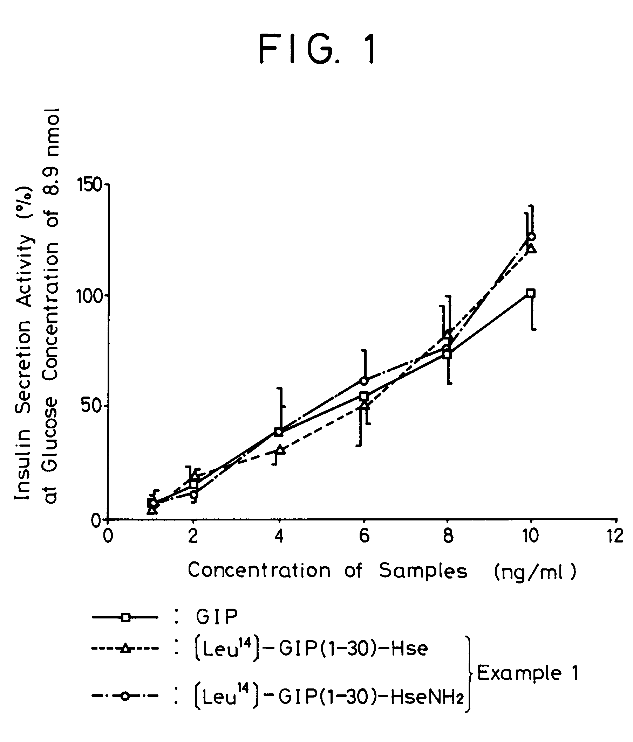

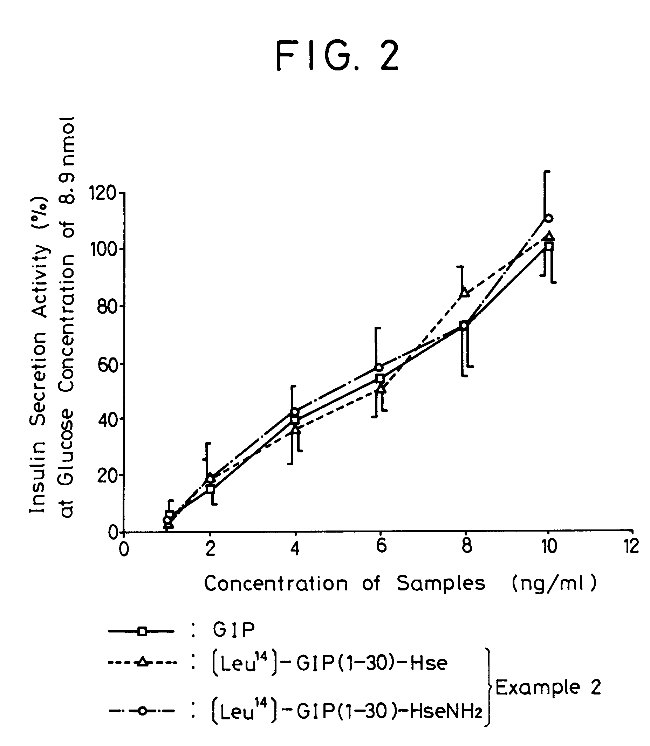

Results are shown in Figs. 1 and 2. As apparently seen from the Figures, each of the GIP analogues according to the invention shows the insulin secretion activity in same level with or higher than GIP.

### Medicine Preparation Example

A solution of the GIP analogue according to the invention [the polypeptide (1)-a] in refined water was aseptically charged into vials, so that each vial contains the polypeptide by 1mg. After lyophilized, the vial was sealed to obtain a dry powdery medicine. The powdery medicine is dissolved in saline or the like for injection purpose, when it shall be used.

For stabilizing the GIP analogue, a human serum albumin or the like can be used.

S E Q U E N C E   L I S T I N G

SEQ ID NO : 1

SEQUENCE TYPE : Amino acid
SEQUENCE LENGTH : 31 Amino acid residues

STRANDNESS : Single
TOPOLOGY : Linear

PROPERTIES : Human GIP (gastric inhibitory polypeptide) analogue

5 10 15
Tyr-Ala-Glu-Gly-Thr-Phe-Ile-Ser-Asp-Tyr-Ser-Ile-Ala-Xaa-Asp-

20 25 30
Lys-Ile-His-Gln-Gln-Asp-Phe-Val-Asn-Trp-Leu-Leu-Ala-Gln-Lys-

homoserine (or homoserine-lactone)

SEQ ID NO : 2

SEQUENCE TYPE : Amino acid
SEQUENCE LENGTH : 31 Amino acid residues

STRANDNESS : Single
TOPOLOGY : Linear

PROPERTIES : Human GIP (gastric inhibitory polypeptide) analogue

5 10 15
Tyr-Ala-Glu-Gly-Thr-Phe-Ile-Ser-Asp-Tyr-Ser-Ile-Ala-Xaa-Asp-

20 25 30
Lys-Ile-His-Gln-Gln-Asp-Phe-Val-Asn-Trp-Leu-Leu-Ala-Gln-Lys-

homoserine (or homoserine-lactone)-amide

SEQ ID NO : 3

SEQUENCE TYPE : Amino acid
SEQUENCE LENGTH : 31 Amino acid residue

STRANDNESS : Single
TOPOLOGY : Linear

PROPERTIES : Human GIP (gastric inhibitory polypeptide) analogue

                    5                   10                  15
Tyr-Ala-Glu-Gly-Thr-Phe-Ile-Ser-Asp-Tyr-Ser-Ile-Ala-Xaa-Asp-

                    20                  25                  30
Lys-Ile-His-Gln-Gln-Asp-Phe-Val-Asn-Trp-Leu-Leu-Ala-Gln-Lys-

                    35                  40
Gly-Lys-Lys-Asn-Asp-Trp-Lys-His-Asn-Ile-Thr-Gln-homoserine

(or homoserine-lactone)


SEQ ID NO : 4

SEQUENCE TYPE : Amino acid
SEQUENCE LENGTH : 31 Amino acid residues
STRANDNESS : Single
TOPOLOGY : Linear

PROPERTIES : Human GIP (gastric inhibitory polypeptide) analogue

                    5                   10                  15
Tyr-Ala-Glu-Gly-Thr-Phe-Ile-Ser-Asp-Tyr-Ser-Ile-Ala-Xaa-Asp-

                    20                  25                  30
Lys-Ile-His-Gln-Gln-Asp-Phe-Val-Asn-Trp-Leu-Leu-Ala-Gln-Lys-

                    35                  40
Gly-Lys-Lys-Asn-Asp-Trp-Lys-His-Asn-Ile-Thr-Gln-homoserine

(or homoserine-lactone)-amide

## Claims (Claims for the following Contracting State(s): AT, BE, CH, DE, DK, FR, GB, GR, IT, LI, LU, NL, SE)

1. A GIP analogue coding an amino acid sequence shown by Formula (1) of 
Tyr-Ala-Glu-Gly-Thr-Phe-Ile-Ser-Asp-Tyr-Ser-Ile-Ala- X -
Asp-Lys-Ile-His-Gln-Gln-Asp-Phe-Val-Asn-Trp-Leu-Leu-Ala-
Gln-Lys- Y
 wherein X is an amino acid residue other than Met, and Y is
a) homoserine (inclusive of homoserine-lactone and indicated by a symbol of "Hse"),
b) homoserine amide (HseNH₂),
c) Gly-Lys-Lys-Asn-Asp-Trp-Lys-His-Asn-Ile-Thr-Gln-Hse or
d) Gly-Lys-Lys-Asn-Asp-Trp-Lys-His-Asn-Ile-Thr-Gln-HseNH₂.

2. A pharmaceutical composition comprising an effective amount of GIP analogue as claimed in Claim 1, and a pharmacologically acceptable carrier or excipient.

## Claims (Claims for the following Contracting State(s): ES)

1. A process for the preparation of a GIP analogue coding an amino acid sequence shown by Formula (1) of 
Tyr-Ala-Glu-Gly-Thr-Phe-Ile-Ser-Asp-Tyr-Ser-Ile-Ala-X-Asp-
Lys-Ile-His-Gln-Gln-Asp-Phe-Val-Asn-Trp-Leu-Leu-Ala-Gln-
Lys-Y
 wherein X is an amino acid residue other than Met, and Y is
a) homoserine (inclusive of homoserine-lactone and indicated by a symbol of "Hse"),
b) homoserine amide (HseNH₂),
c) Gly-Lys-Lys-Asn-Asp-Trp-Lys-His-Asn-Ile-Thr-Gln-Hse-or
d) Gly-Lys-Lys-Asn-Asp-Trp-Lys-His-Asn-Ile-Thr-Gln-HseNH₂,
which process is characterized by comprising cloning a suitable double-stranded DNA to insert the same into a vector or plasmid and in this way transform a host, such as Escherichia coli, which shall be cultured to express the GIP analogue in large amount, followed by isolation and purification thereof.

2. A process for the preparation of a pharmaceutical composition based on GIP analogue, such as defined in claim 1, which process is characterized by comprising mixing a pharmaceutically effective amount of said GIP analogue with a pharmacologically acceptable carrier or excipient.

## Patentansprüche (Patentansprüche für folgende(n) Vertragsstaat(en): AT, BE, CH, DE, DK, FR, GB, GR, IT, LI, LU, NL, SE)

1. GIP-Analogon, das für eine Aminosäureseguenz kodiert, die durch die Formel (1) dargestellt wird: 
Tyr-Ala-Glu-Gly-Thr-Phe-Ile-Ser-Asp-Tyr-Ser-Ile-Ala-X-
Asp-Lys-Ile-His-Gln-Gln-Asp-Phe-Val-Asn-Trp-Leu-Leu-Ala-
Gln-Lys-Y,
 in der X ein von Met verschiedener Aminosäurerest ist und Y
a) Homoserin (einschließlich Homoserin-Lacton und durch das Symbol "Hse" angezeigt),
b) Homoserinamid (HseNH₂),
c) Gly-Lys-Lys-Asn-Asp-Trp-Lys-His-Asn-Ile-Thr-Gln-Hse oder
d) Gly-Lys-Lys-Asn-Asp-Trp-Lys-His-Asn-Ile-Thr-Gln-HseNH₂
ist.

2. Pharmazeutische Zusammensetzung, umfassend eine wirksame Menge von in Anspruch 1 beanspruchtem GIP-Analogon und einen pharmakologisch annehmbaren Träger oder Hilfsstoff.

## Patentansprüche (Patentansprüche für folgende(n) Vertragsstaat(en): ES)

1. Verfahren zur Herstellung eines GIP-Analogons, das für eine Aminosäuresequenz kodiert, die durch die Formel (1) dargestellt wird: 
Tyr-Ala-Glu-Gly-Thr-Phe-Ile-Ser-Asp-Tyr-Ser-Ile-Ala-X-
Asp-Lys-Ile-His-Gln-Gln-Asp-Phe-Val-Asn-Trp-Leu-Leu-Ala-
Gln-Lys-Y,
 in der X ein von Met verschiedener Aminosäurerest ist und Y
a) Homoserin (einschließlich Homoserin-Lacton und durch das Symbol "Hse" angezeigt),
b) Homoserinamid (HseNH₂),
c) Gly-Lys-Lys-Asn-Asp-Trp-Lys-His-Asn-Ile-Thr-Gln-Hse oder
d) Gly-Lys-Lys-Asn-Asp-Trp-Lys-His-Asn-Ile-Thr-Gln-HseNH₂,
ist,
wobei das Verfahren dadurch gekennzeichnet ist, daß es das Klonieren einer geeigneten doppelsträngigen DNA umfaßt, um dieselbe in einen Vektor oder ein Plasmid zu insertieren und auf diesem Weg einen Wirt, wie beispielsweise Escherichia coli, zu transformieren, welcher kultiviert werden soll, um das GIP-Analogon in großer Menge zu exprimieren, gefolgt von der Isolierung und Reinigung desselben.

2. Verfahren zur Herstellung einer pharmazeutischen Zusammensetzung, die auf einem wie in Anspruch 1 definierten GIP-Analogon basiert, wobei das Verfahren dadurch gekennzeichnet ist, daß es das Mischen einer pharmazeutisch wirksamen Menge des GIP-Analogons mit einem pharmakologisch annehmbaren Träger oder Hilfsstoff umfaßt.

## Revendications (Revendications pour l'(les) Etat(s) contractant(s) suivant(s): AT, BE, CH, DE, DK, FR, GB, GR, IT, LI, LU, NL, SE)

1. Analogue du GIP code par la séquence d'acides aminés répondant à la formule (1) suivante 
Tyr-Ala-Glu-Gly-Thr-Phe-Ile-Ser-Asp-Tyr-Ser-Ile-Ala- X -
Asp-Lys-Ile-His-Gln-Gln-Asp-Phe-Val-Asn-Trp-Leu-Leu-Ala-
Gln-Lys- Y
 dans laquelle X est un résidu d'acide aminé autre que la Met, et Y est
a) une homosérine (y compris l'homosérine-lactone et désignée par le symbole "Hse"),
b) un homosérine amide (HseNH₂),
c) Gly-Lys-Lys-Asn-Asp-Trp-Lys-His-Asn-Ile-Thr-Gln-Hse
ou
d) Gly-Lys-Lys-Asn-Asp-Trp-Lys-His-Asn-Ile-Thr-Gln-HseNH₂.

2. Composition pharmaceutique comprenant un analogue du GIP, tel que revendiqué dans la revendication 1, en quantité efficace, et un véhicule ou excipient pharmacologiquement acceptable.

## Revendications (Revendications pour l'(les) Etat(s) contractant(s) suivant(s): ES)

1. Procédé de préparation d'un analogue de GIP codé par la séquence d'acides aminés répondant à la Formule (1) suivante 
Tyr-Ala-Glu-Gly-Thr-Phe-Ile-Ser-Asp-Tyr-Ser-Ile-Ala-X-Asp-
Lys-Ile-His-Gln-Gln-Asp-Phe-Val-Asn-Trp-Leu-Leu-Ala-Gln-
Lys-Y
 dans laquelle X est un résidu d'acide aminé autre que la Met, et Y est
a) une homosérine (y compris l'homosérine-lactone et désignée par le symbole "Hse"),
b) un homosérine amide (HseNH₂),
c) Gly-Lys-Lys-Asn-Asp-Trp-Lys-His-Asn-Ile-Thr-Gln-Hse-
ou
d) Gly-Lys-Lys-Asn-Asp-Trp-Lys-His-Asn-Ile-Thr-Gln-HseNH₂,
lequel procédé étant caractérisé en ce qu'il comprend le clonage d'un ADN double brin convenable destiné à être inséré dans un vecteur ou plasmide de manière à transformer un hôte, tel que *Escherichia coli*, qui sera mis en culture pour exprimer l'analogue du GIP en grande quantité, et enfin l'isolement et la purification de ce dernier.

2. Procédé de préparation d'une composition pharmaceutique à base d'un analogue du GIP, tel que défini dans la revendication 1, lequel procédé étant caractérisé en ce qu il comprend le mélange d'une quantité pharmaceutiquement efficace dudit analogue du GIP avec un véhicule ou excipient pharmacologiquement acceptable.
